# FASCICULE DE BREVET EUROPEEN

(11) **EP 0 779 796 B1**
(45) Date de publication et mention de la délivrance du brevet: **31.03.1999**
(21) Numéro de dépôt: 95925895.5
(22) Date de dépôt: 17.07.1995
(51) Int. Cl.: A61B 17/70

(54) **DISPOSITIF D'OSTEOSYNTHESE RACHIDIENNE**
OSTEOSYNTHESEVORRICHTUNG FÜR DIE WIRBELSÄULE
SPINAL OSTEOSYNTHESIS DEVICE

(30) Priorité: 18.07.1994 FR 9409125
(43) Date de publication de la demande: 25.06.1997
(73) Titulaire: SURGICRAFT LIMITED, Redditch Worcs. B97 6HF (GB)
(72) Inventeur: ONIMUS, Michel, F-25000 Besançon (FR); GANGLOFF, Serge, F-25000 Besançon (FR)
(74) Mandataire: Maureau, Philippe
(86) Numéro de dépôt international: FR9500955
(87) Numéro de publication internationale: WO9602199

(56) Documents cités:
- EP-A- 0 553 424
- EP-A- 0 553 782
- WO-A-90/04948
- WO-A-91/11967
- DE-A- 4 035 548
- FR-A- 2 704 137
- GB-A- 2 178 323
- GB-A- 2 254 370
- US-A- 3 651 803
- US-A- 5 002 542

## Description

La présente invention concerne un dispositif d'ostéosynthèse rachidienne.

Un rachis peut présenter une déformation longitudinale ou une instabilité résultant d'une usure discale, qui peuvent rendre nécessaire d'implanter un dispositif d'ostéosynthèse. Ce dispositif est fixé à plusieurs vertèbres consécutives, par voie postérieure, et vient redresser et étayer le rachis.

Il existe des dispositifs, connus par exemple par les demandes de brevet n° GB 2 178 323, EP 0 553 424, EP 0 553 782, US 5 002 542 ou WO 90/04948, comprenant:
- un ou plusieurs éléments rigides d'étayage, généralement constitués par des tiges métalliques venant se positionner de part et d'autre des apophyses épineuses des vertèbres, ou par des plaques ou cadres rectangulaires venant s'engager autour de plusieurs apophyses épineuses consécutives ;
- des moyens de fixation de ces éléments d'étayage au rachis, le plus fréquemment constitués par des vis ou crochets pédiculaires, et
- des éléments intermédiaires d'assemblage du ou des éléments d'étayage et des moyens de fixation.

La mise en place de certains de ces matériels n'est pas toujours très facile à réaliser. En effet, le positionnement des éléments d'étayage et le perçage des trous venant recevoir les vis pédiculaires peuvent constituer des opérations particulièrement délicates à réaliser, d'autant plus que les tissus entourant la colonne vertébrale sont très vascularisés et saignent beaucoup. Il en résulte que la durée de l'intervention est importante, ce qui n'est guère souhaitable pour le patient.

En outre, les matériels existants comprennent généralement de nombreuses pièces de petites dimensions, devant être successivement mises en place. Ces assemblages sont longs et peuvent être difficiles à réaliser, ce qui contribue à prolonger l'intervention. Les dimensions réduites de ces pièces peuvent obliger le praticien à des interventions manuelles, non souhaitables au plan de l'asepsie.

On connaît notamment un dispositif dans lequel l'élément d'étayage, en forme de cadre rectangulaire, est assemblé aux vis assurant sa fixation au moyen d'un ou plusieurs ponts transversaux. Chaque pont a une forme en oméga, c'est-à-dire comprend deux ailes latérales percées de trous pour le passage des vis et une partie centrale incurvée servant de point d'appui latéral aux tiges constituant les côtés longitudinaux du cadre d'étayage. L'assemblage de ces tiges à un pont est assuré par deux plaquettes latérales venant enserrer les tiges entre elles et le pont. Chacune de ces plaquettes comprend une partie centrale incurvée, venant entourer la tige, une aile latérale percée d'un trou qui se superpose au trou du pont, pour le passage de la vis pédiculaire, et une patte latérale, située à l'opposé de l'aile précitée, venant s'insérer dans un trou récepteur aménagé dans la partie centrale incurvée du pont.

Pour son implantation, ce dispositif implique successivement :
- de positionner provisoirement le ou les ponts puis le cadre d'étayage sur le rachis, dans leur position d'implantation, au moyen de fils métalliques sous-lamaires,
- de repérer puis percer les trous des vis dans les pédicules,
- de mettre les plaquettes précitées en place sur les ponts,
- de maintenir les trous du ou des ponts et des plaquettes en face des trous percés dans l'os, et
- de mettre les vis pédiculaires en place.

Ces différentes opérations rendent la pose de ce dispositif complexe et difficile, compte tenu des conditions opératoires précitées. En particulier, le perçage des trous peut être relativement imprécis et l'engagement des vis en eux, après positionnement adéquat des ponts, cadre et plaquettes précités, peut être relativement difficile, surtout en cas de vertèbre(s) déviée(s).

La présente invention vise à remédier à l'ensemble de ces inconvénients, en fournissant un dispositif d'ostéosynthèse rachidienne nettement plus facile et rapide à implanter que les dispositifs existants. En particulier, ce dispositif doit pouvoir être implanté uniquement à l'aide de pinces, selon la technique dite "no touch", donc sans aucun contact entre les pièces implantées et les mains du praticien.

Le dispositif qu'elle concerne est du type comprenant un ou plusieurs éléments rigides d'étayage, des vis ou moyens d'ancrage osseux similaires, et des éléments intermédiaires d'assemblage de cet élément d'étayage et de ces vis, la tête de chaque vis présentant une tige filetée faisant saillie à l'opposé du corps de la vis, et un épaulement formant une surface d'appui perpendiculaire à l'axe de cette tige filetée, chaque élément intermédiaire étant destiné à être engagé sur ladite tige et à être serré contre l'épaulement par un écrou vissé sur la tige.

Selon l'invention :
- chaque élément intermédiaire comprend deux plaquettes articulées l'une à l'autre par l'une de leurs extrémités, présentant des trous oblongs dans leur autre extrémité, ces plaquettes pouvant recevoir l'élément d'étayage entre elles et enserrer cet élément avec des frottements, ces frottements étant suffisants pour que l'élément intermédiaire ne puisse, avant implantation, pivoter sous son propre poids par rapport à l'élément d'étayage mais insuffisants pour empêcher tout mouvements de l'élément intermédiaire par rapport à l'élément d'étayage.

Les vis sont tout d'abord implantées dans les vertèbres.

Les éléments intermédiaires sont mis en place sur le ou les éléments d'étayage, en étant positionnés de la manière la plus adéquate en fonction de la position des vis, puis l'ensemble est introduit dans la cavité.

Lors de cette introduction, les éléments intermédiaires conservent leur position par rapport à l'élément d'étayage, du fait des frottements précités. Lorsque l'élément d'étayage est placé en position définitive, les éléments intermédiaires se trouvent par conséquent positionnés à proximité immédiate des vis. Ils peuvent alors être facilement saisis à l'aide de pinces pour être engagés, par un mouvement de pivotement limité par rapport à l'élément d'étayage, sur les tiges filetées que comprennent les têtes des vis.

Les écrous sont mis en place et serrés pour assurer l'immobilisation de l'ensemble, une fois tous les éléments intermédiaires engagés sur les tiges filetées.

Les trous oblongs des éléments intermédiaires permettent de corriger d'éventuels défauts de positionnements des vis.

Ainsi, le dispositif selon l'invention permet d'implanter les vis en premier, immédiatement après le perçage des trous dans l'os, et d'assurer une mise en place simple, facile et rapide du ou des éléments d'étayage et des éléments intermédiaires, avec accomplissements de gestes minimes et sans aucun contact des mains du praticien avec les pièces implantées.

Ce dispositif peut, en outre, être réalisé en titane, ce qui n'est pas le cas du dispositif existant comprenant des fils sous-lamaires, ce matériau ne pouvant être torsadé dans de bonnes conditions.

Avantageusement, le dispositif comprend des moyens permettant d'assurer des frottements sensiblement constants, quelles que soient les tolérances de fabrication des plaquettes et de leur axe d'assemblage.

Pour sa bonne compréhension, l'invention est à nouveau décrite ci-dessous en référence au dessin schématique annexé représentant, à titre d'exemple non limitatif, une forme de réalisation préférée du dispositif d'ostéosynthèse rachidienne qu'elle concerne.
La figure 1 est une vue en perspective d'une vis pédiculaire, d'une partie de l'élément d'étayage et d'un élément intermédiaire d'assemblage de cet élément d'étayage et de cette vis, que ce dispositif comprend ;
la figure 2 est une vue en plan de l'élément intermédiaire, avant assemblage des deux plaquettes articulées qui le constituent ;
la figure 3 est une vue en coupe de l'élément intermédiaire, en cours d'engagement sur la vis ;
la figure 4 est une vue en perspective de ce dispositif, après implantation sur un rachis, et
la figure 5 en est une vue similaire à la figure 3, après engagement sur la vis.

La figure 4 représente un dispositif 1 d'ostéosynthèse rachidienne, fixé à plusieurs vertèbres consécutives 2, par voie postérieure.

Dans l'exemple représenté, ce dispositif comprend un cadre rigide d'étayage 3, des vis pédiculaires 4 d'ancrage osseux, comportant des écrous 5, et des éléments intermédiaires 6 permettant d'assurer l'assemblage de ce cadre rigide 3 et de ces vis 4 les uns aux autres, ces éléments intermédiaires 6 étant serrés entre les têtes des vis et les écrous 5.

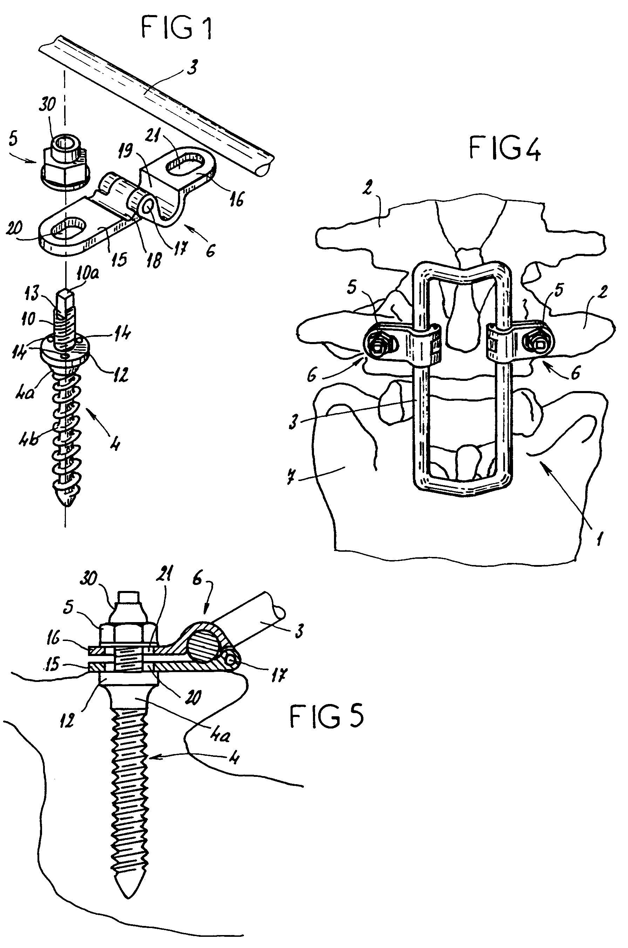

Les figures 1,2,3 et 5 montrent plus précisément une vis pédiculaire 4, un écrou 5, un élément intermédiaire 6 et une portion du cadre 3, sous différents angles.

Ainsi que cela est visible sur ces figures :
- la tête 4a de chaque vis 4 présente une tige filetée 10 pouvant recevoir l'écrou 5, cette tige 10 faisant saillie à l'opposé du corps 4b de la vis 4, parallèlement à celui-ci ;
- la tête 4a présente un épaulement 12 formant une surface d'appui perpendiculaire à l'axe de cette tige filetée 10 ;
- l'extrémité libre 10a de la tige 10 présente une section transversale carrée et deux méplats 13 diamétralement opposés ;
- la tête 4a de la vis 4 présente trois trous 14 régulièrement répartis sur sa circonférence ;
- chaque élément intermédiaire 6 comprend deux plaquettes 15,16 articulées l'une à l'autre par l'une de leurs extrémités, autour d'un axe 17 ;
- les plaquettes 15 et 16 comprennent des évidements 18 et 19 leur permettant de venir enserrer la partie du cadre 3 sur lesquels elles peuvent être engagées ;
- les plaquettes comprennent des trous 20 et 21 de forme oblongue venant en correspondance l'un avec l'autre lorsque elles enserrent le cadre 3 ; et
- l'écrou 5 est prolongé, en partie supérieure, par une jupe métallique déformable 30, susceptible de venir en correspondance des méplats 13 que comprend l'extrémité libre de la tige filetée 10a, lorsque cet écrou 5 est serré.

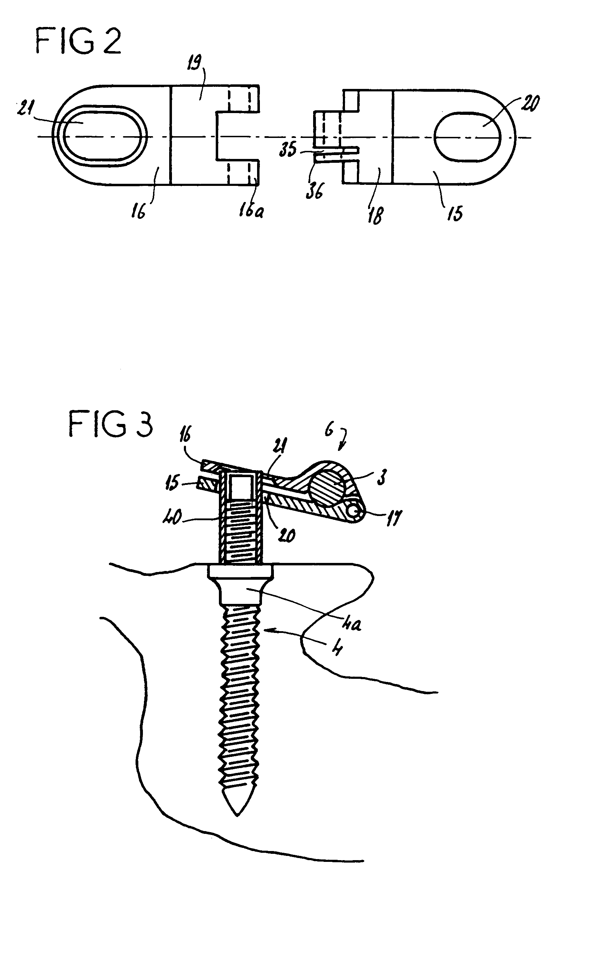

La figure 2 montre plus particulièrement que la plaquette 15 comprend une fente 35 aménagée perpendiculairement à l'axe de l'alésage destiné à recevoir l'axe 17, cette fente délimitant un oeillet 36 rattaché à la plaquette 15 par un pont de matière. Il apparaît à la figure 2 que ce pont est déformé de telle sorte que l'oeillet 36 ne soit pas perpendiculaire audit axe, mais fasse saillie vers l'extérieur (c'est-à-dire vers le bas de la figure 2), au delà de la patte d'assemblage 16a de la plaquette 16. Il en résulte que le pont est élastiquement déformé lors de l'assemblage des plaquettes 15 et 16, et que l'oeillet 36 est appliqué contre la face interne de la patte 16a, générant des frottements qui s'opposent au pivotement des plaquettes 15 et 16 autour de l'axe 17.

Les figures 2 et 3 montrent que la plaquette 16 est chanfreinée au niveau du trou 21, dans sa face tournée du côté de la vis 4. Un même chanfrein peut être aménagé autour du trou 20.

La figure 3 montre également qu'un manchon 40 peut être mis en place sur la tige filetée 10 pour protéger le filet de celle-ci.

En outre, la face de la plaquette 16 contre laquelle l'écrou 5 vient en appui présente des stries.

Les plaquettes 15 et 16 peuvent, dans leur position relative représentée à la figure 1, recevoir le cadre 3 entre elles, et, dans leur position relative représentée aux figures 3 à 5, enserrer ledit cadre 3, avec venue des trous 20 et 21 en correspondance l'un de l'autre.

Les frottements résultant de l'application de l'oeillet 36 sur la patte 16a de la plaquette 16 sont prévus de manière à être suffisants pour que l'élément intermédiaire 6 ne puisse, avant implantation, pivoter sous son propre poids par rapport au cadre 3 mais insuffisants pour empêcher tout mouvements de l'élément 6 par rapport au cadre 3.

Cette application de l'oeillet 36 sur la patte 16a permet d'assurer des frottements sensiblement constants, quelles que soient les tolérances de fabrication des plaquettes 15,16 et de l'axe 17.

En pratique, les vis 4 sont tout d'abord implantées dans les vertèbres 2, avec les manchons 40 engagés sur les tiges filetées 10.

Les éléments intermédiaires 6 sont mis en place sur le cadre 3, en étant positionnés de la manière la plus adéquate en fonction de la position des vis 4, puis l'ensemble est introduit dans la cavité.

Lors de cette introduction, les éléments 6 conservent leur position par rapport au cadre 3, du fait des frottements précités. Lorsque le cadre 3 est placé en position définitive, les éléments 6 se trouvent positionnés à proximité immédiate des vis 4. Ils peuvent alors être facilement saisis à l'aide de pinces pour être engagés, ainsi que le montre la figure 3, sur les manchons 40, selon un mouvement de pivotement limité par rapport au cadre 3.

Les chanfreins des plaquettes 16 facilitent cet engagement, et les manchons 40 permettent de protéger les filets des tiges 10.

Une fois l'ensemble des éléments 6 engagé sur les tiges filetées 10, les manchons 40 sont retirés et les écrous 5 sont mis en place et serrés pour assurer l'immobilisation des éléments 6 entre les épaulements 12 et les écrous 5.

Les trous oblongs 20 et 21 permettent de corriger d'éventuels défauts de positionnements des vis 4.

Les stries des plaquettes 16 favorisent l'immobilisation des écrous 5 en rotation.

La jupe métallique 30 est destinée à être sertie sur les méplats 13 de la tige 10, de manière à assurer une parfaite immobilisation en rotation de l'écrou 5 par rapport à la vis 4. Cette immobilisation élimine tout risque de desserrage sous l'effet des contraintes répétées que subit l'assemblage cadre 3-vis pédiculaires 4.

Les trous 14 permettent l'engagement de tétons complémentaires aménagés dans l'extrémité de l'instrument ancillaire (non représenté) permettant la mise en place des vis 4 dans l'os. L'extrémité libre 10a des tiges 10 permet le maintien en rotation, à l'aide d'un instrument approprié, des vis 4 au moment du serrage des écrous 5.

Ainsi, le dispositif selon l'invention permet d'implanter les vis 4 en premier, immédiatement après le perçage des trous dans l'os, et d'assurer une mise en place simple, facile et rapide du cadre 3 et des éléments intermédiaires 6, avec accomplissements de gestes minimes et sans aucun contact des mains du praticien avec les pièces implantées.

Il va de soi que l'invention n'est pas limitée à la forme de réalisation décrite ci-dessus à titre d'exemple mais qu'elle en embrasse, au contraire, toutes les variantes de réalisation. Ainsi, d'autres moyens que l'oeillet 36 peuvent être envisagés pour assurer les frottements adéquats entre les plaquettes 15 et 16, par exemple des aspérités de surface sur l'axe 17 ou les faces adjacentes des plaquettes.

## Revendications

1. Dispositif d'ostéosynthèse rachidienne, du type comprenant un ou plusieurs éléments rigides d'étayage (3), des vis (4) ou moyens d'ancrage osseux similaires, et des éléments intermédiaires (6) d'assemblage de cet élément d'étayage (3) et de ces vis (4), la tête (4a) de chaque vis (4) présentant une tige filetée (10) faisant saillie à l'opposé du corps (4b) de la vis (4), et un épaulement (12) formant une surface d'appui perpendiculaire à l'axe de cette tige filetée (10), chaque élément intermédiaire (6) étant destiné à être engagé sur ladite tige (10) et à être serré contre l'épaulement (12) par un écrou (5) vissé sur la tige (10), dispositif caractérisé en ce que :
- chaque élément intermédiaire (6) comprend deux plaquettes (15,16) articulées l'une à l'autre par l'une de leurs extrémités, présentant des trous oblongs (20,21) dans leur autre extrémité, ces plaquettes (15,16) pouvant recevoir l'élément d'étayage (3) entre elles et enserrer cet élément (3) avec des frottements, ces frottements étant suffisants pour que l'élément intermédiaire (6) ne puisse, avant implantation, pivoter sous son propre poids par rapport à l'élément d'étayage (3) mais insuffisants pour empêcher tout mouvements de l'élément intermédiaire (6) par rapport à l'élément d'étayage (3).

2. Dispositif selon la revendication 1, caractérisé en ce qu'il comprend des moyens (36) permettant d'assurer des frottements sensiblement constants, quelles que soient les tolérances de fabrication des plaquettes (15,16) et de leur axe d'assemblage (17).

3. Dispositif selon la revendication 2, caractérisé en ce que l'une des plaquettes (15) comprend une fente (35) aménagée perpendiculairement à l'axe de l'alésage destiné à recevoir l'axe (17), cette fente délimitant un oeillet (36) rattaché à la plaquette (15) par un pont de matière, l'oeillet (36) n'étant pas perpendiculaire à l'axe (17) mais faisant saillie vers l'extérieur, au-delà de la patte d'assemblage (16a) de la plaquette (16), et le pont étant, après assemblage des plaquettes (15,16), déformé de telle sorte que l'oeillet 36 est appliqué contre la patte d'assemblage (16a) correspondante de l'autre plaquette (16), générant les frottements précités.

4. Dispositif selon l'une des revendications 1 à 3, caractérisé en ce qu'au moins une des plaquettes (15,16) est chanfreinée au niveau de son trou oblong (20,21), dans sa face tournée du côté de la vis (4).

5. Dispositif selon l'une des revendications 1 à 4, caractérisé en ce que des manchons (40) sont mis en place sur les tiges filetées (10) pour protéger le filet de celles-ci lors de l'engagement des éléments intermédiaires (6) sur elles.

6. Dispositif selon l'une des revendications 1 à 5, caractérisé en ce que les faces des plaquettes (16) contre lesquelles les écrous (5) viennent en appui présentent des surfaces favorisant l'immobilisation des écrous (5) en rotation, par exemple des stries ou un moletage.

7. Dispositif selon l'une des revendication 1 à 6, caractérisé en ce que chaque écrou (5) est prolongé, en partie supérieure, par une jupe métallique déformable (30), et en ce que chaque tige filetée (10) des vis (4) comprend des méplats (13) venant en correspondance de cette jupe (30) lorsque l'écrou (5) est serré.

## Claims

1. Spinal osteosynthesis device, of the type comprising one or more rigid support elements (3), screws (4) or similar bone anchoring means, and intermediate elements (6) for connecting this support element (3) and these screws (4), the head (4a) of each screw (4) having a threaded shank (10) projecting in the opposite direction to the body (4b) of the screw (4), and a shoulder (12) forming a bearing surface perpendicular to the axis of this threaded shank (10), each intermediate element (6) being intended to be engaged on the said shank (10) and to be clamped against the shoulder (12) by a nut (5) screwed onto the shank (10), a device characterised in that:
- each intermediate element (6) comprises two brackets (15, 16) articulated on each other by one of their ends, having oblong holes (20, 21) in their other end, these brackets (15, 16) being able to receive the support element (3) between them and to grip this element (3) with friction, this friction being sufficient for the intermediate element (6) not to be able, after fitting, to pivot under its own weight with respect to the support element (3) but insufficient to prevent any movement of the intermediate element (6) with respect to the support element (3).

2. Device according to Claim 1, characterised in that in that it comprises means (36) for providing substantially constant friction, whatever the manufacturing tolerances on the brackets (15, 16) and their connecting pin (17).

3. Device according to Claim 2, characterised in that one of the brackets (15) comprises a slot (35) provided perpendicular to the axis of the bore intended to receive the pin (17), this slot delimiting an eye (36) attached to the bracket (15) by a bridge of material, the eye (36) not being perpendicular to the pin (17) but projecting outwards, beyond the connecting lug (16a) on the plate (16), and the bridge being, after connection of the brackets (15, 16), deformed so that the eye 36 is applied against the corresponding assembly lug (16a) on the other bracket (16), generating the aforementioned friction.

4. Device according to one of Claims 1 to 3, characterised in at least one of the brackets (15, 16) is bevelled at its oblong hole (20, 21), in its face turned towards the screw (4).

5. Device according to one of Claims 1 to 4, characterised in that sleeves (40) are fitted on the threaded shanks (10) in order to protect the thread thereon when the intermediate elements (6) are engaged on them.

6. Device according to one of Claims 1 to 5, characterised in that the faces of the brackets (16) against which the nuts (5) come into abutment have surfaces assisting the immobilisation of the nuts (5) with respect to rotation, for example serrations or knurling.

7. Device according to one of Claims 1 to 6, characterised in that each nut (5) is extended, at the top, by a deformable metallic skirt (30), and in that each threaded shank (10) on the screws (4) comprises flats (13) coming to coincide with this skirt (30) when the nut (5) is tightened.

## Patentansprüche

1. Vorrichtung zur Osteosynthese von Wirbeln, vom Typ, der ein oder mehrere steife Stützelemente (3), Schrauben (4) oder ähnliche Mittel zum Verankern in Knochen und Verbindungselemente (6) zum Zusammenbauen dieser Stützelemente (3) und dieser Schrauben (4) umfaßt, wobei der Kopf (4a) jeder Schraube (4) eine Gewindestange (10), die gegenüber zu dem Körper (4b) der Schraube (4) hervorsteht, und eine Schulter (12) aufweist, die eine senkrecht zu der Achse dieser Gewindestange (10) angeordnete Auflagefläche bildet, wobei jedes Verbindungselement (6) dazu vorgesehen ist, auf der Gewindestange (10) eingesetzt und gegen die Schulter (12) durch eine auf die Gewindestange (10) geschraubte Mutter (5) festgeklemmt zu werden, wobei die Vorrichtung dadurch gekennzeichnet ist, daß:
- jedes Verbindungselement (6) zwei Platten (15, 16) umfaßt, wobei die eine an der anderen an ihren Enden angelenkt ist, die Langlöcher (20, 21) in ihren anderen Enden aufweisen, wobei diese Platten (15, 16) das Stützelement (3) zwischen sich aufnehmen können und dieses Element (3) mit Reibung festhalten, wobei diese Reibung ausreichend ist, so daß das Verbindungselement (6) sich nicht vor der Implantation unter seinem eigenen Gewicht im Verhältnis zu dem Stützelement (3) drehen kann, aber nicht ausreichend ist, um alle Bewegungen des Verbindungselementes (6) im Verhältnis zu dem Stützelement (3) zu verhindern.

2. Vorrichtung nach Anspruch 1, dadurch gekennzeichnet, daß sie Mittel (36) umfaßt, die ein Gewährleisten einer wahrnehmbaren konstanten Reibung ermöglicht, welche die Fabrikationstoleranzen der Platten (15, 16) und ihrer Zusammenbauachse (17) ausgleicht.

3. Vorrichtung nach Anspruch 2, dadurch gekennzeichnet, daß eine der Platten (15) einen Schlitz (35) umfaßt, der senkrecht zu der Achse der für das Aufnehmen der Achse (17) bestimmten Bohrung angeordnet ist, wobei dieser Schlitz eine Öse (36) begrenzt, die an der Platte (15) an eine Materialbrücke angrenzt, wobei die Öse (36) nicht senkrecht zu der Achse (17) ist, aber nach außen über die Zusammenbauhalter (16a) der Platte (16) hervorsteht, und die Brücke nach dem Zusammenbauen der Platten (15, 16) derart deformiert ist, daß die Öse (36) an dem Zusammenbauhalter (16a) anliegt, der mit der anderen Platte (16) verbunden ist, was die vorgenannte Reibung erzeugt.

4. Vorrichtung nach einem der Ansprüche 1 bis 3, dadurch gekennzeichnet, daß mindestens eine der Platten (15, 16) auf der Höhe ihres Langloches (20, 21) an seiner der der Schraube (4) zugewandten Seite angefast ist.

5. Vorrichtung nach einem der Ansprüche 1 bis 4, dadurch gekennzeichnet, daß Hülsen (40) auf den Gewindestangen (10) positioniert sind, um das Gewinde dieser während des Eingriffes der Verbindungselemente (6) auf ihnen zu schützen.

6. Vorrichtung nach einem der Ansprüche 1 bis 5, dadurch gekennzeichnet, daß die Seiten der Platten (16), gegen die die Muttern (5) anliegen, Oberflächen, die das Festlegen der Muttern (5) hinsichtlich der Rotation fördern, beispielsweise Rillen oder eine Riffelung, aufweisen.

7. Vorrichtung nach einem der Ansprüche 1 bis 6, dadurch gekennzeichnet, daß jede Mutter (5) im oberen Teil durch eine metallische deformierbare Schürze (30) verlängert ist, und daß jede Gewindestange (10) der Schrauben (4) Abflachungen (13) aufweist, die sich entsprechend zu dieser Schürze (30) erstrekken, wenn die Mutter (5) festgeklemmt ist.
